# FASCICULE DE BREVET EUROPEEN

(11) **EP 1 093 359 B1**
(45) Date de publication et mention de la délivrance du brevet: **25.09.2002**
(21) Numéro de dépôt: 99922254.0
(22) Date de dépôt: 03.06.1999
(51) Int. Cl.: A61K 7/48, A61K 47/42, C07K 14/47

(54) **PROCEDE DE PREPARATION DE FIBRILLINE A PARTIR DE CNIDAIRES, ET COMPOSITIONS OBTENUES UTILISABLES EN COSMETIQUES**
VERFAHREN ZUR GEWINNUNG VON FIBRILLIN AUS CNIDARIA UND ENTSPRECHENDE KOSMETISCHE ZUSAMMENSETZUNGEN
METHOD FOR PREPARING FIBRILLIN FROM CNIDARIA, AND RESULTING COMPOSITIONS USEFUL IN COSMETICS

(30) Priorité: 05.06.1998 FR 9807078
(43) Date de publication de la demande: 25.04.2001
(73) Titulaire: JAVENECH (Société Anonyme), 35133 Fougères (FR)
(72) Inventeur: RANSON, Michèle, F-75017 Paris (FR); DAVID, Marc, F-35300 Fougères (FR)
(74) Mandataire: L'Helgoualch, Jean
(86) Numéro de dépôt international: FR9901306
(87) Numéro de publication internationale: WO99063964

(56) Documents cités:
- FR-A- 2 714 063
- REBER-MULLER S ET AL., : "An extracellular matrix protein of jellyfish homologous to mammalian fibrillins forms different fibrils depending on the life stage of the animal" DEVELOPMENTAL BIOLOGY , vol. 169, no. 2, 1995, page 662-672 XP002117293 cité dans la demande
- DATABASE WPI Week 7739 Derwent Publications Ltd., London, GB; AN 77-70092 XP002117298 & SU 534 692 A (LENGD REFRIDGE IND), 21 janvier 1977 (1977-01-21)
- HANDFORD ET AL.,: "The Calcium Binding Properties and Molecular Organization of Epidermal Growth Factor-like Domains in Human Fibrillin-1" JOURNAL OF BIOLOGICAL CHEMISTRY, vol. 270, no. 12, 1995, page 6751-6756 XP002117294
- WERTH V P ET AL., : "UVB irradiation alters cellular responses to cytokines: Role in extracellular matrix gene expression." JOURNAL OF INVESTIGATIVE DERMATOLOGY, vol. 108 (3), 1997, page 290-294 XP002117295
- BERNSTEIN E F ET AL., : "Evaluation of sunscreens with various sun protection factors in a new transgenic mouse model of cutaneous photoaging that measures elastin promoter activation. " JOURNAL OF THE AMERICAN ACADEMY OF DERMATOLOGY, vol. 37 (5 PART 1), 1 novembre 1997 (1997-11-01), page 725-729 XP002117296

## Description

La présente invention concerne une protéine dermique, la fibrilline, présentant une composition déterminée en acides aminés, un procédé de préparation de fibrilline d'origine marine ayant cette composition, et plus particulièrement un procédé de préparation de fibrilline à partir de cnidaires, notamment de méduses, ainsi que des compositions cosmétiques et pharmaceutiques la contenant.

La fibrilline est une macromolécule non collagénique du tissu conjonctif qui est normalement insoluble dans ces tissus et a été purifiée à partir de milieu de culture de fibroblaste [« Fibrillin, A new 350-kD Glycoprotein, is a Component of Extracellular Microfibrils » *Lynn Y. Sakai and all ; The Journal of Cell Biology, Vol. 103 (N°6, Pt.1) Dec. 1986 2499-2509*]. Son altération est à l'origine du syndrome de Marfan.

La fibrilline est un des constituants majeur des microfibrilles du derme. Ces microfibrilles sont particulièrement abondantes dans les tissus élastiques où elles forment un échafaudage sur lequel l'élastine se dépose.

Cette fonction d'accrochage de la fibrilline existe également dans d'autres tissus [« Extraction of extendable beaded structures and their identification as fibrillin-containing extracellular matrix microfibrils » *Kenne, D.R. and all ; J. Histochem. Cytochem. 39, 441-449*].

La fibrilline a une structure complexe comprenant une quarantaine de motif EGF (Epidermal Growth Factor) organisés en tandem [« Fibrillin binds calcium and is coded by cDNAs that reveal a multidomain structure and alternatively spliced exons at the 5' end » *Corson, G.M. and all ; Genomics 17, 476-484*].

De plus, on sait que la fibrilline en fixant le LTBP-1 (Latent Transforming Growth Factor-Binding Protein-1) peut stocker du TGF-β (Transforming Growth Factor β 1 and 2). Ce dernier est reconnu pour favoriser la formation de la Matrice Extracellulaire et inhiber sa dégradation [« Fibrillin-Containing Microfibrils of Normal and Regenerating Skin content Latent Transforming Growth Factor-Binding Protein-1 (LTBP-1) and are a Repository for Latent TGF-β1 » *M. Raghunath and all, J. Invest. Dermatol. 110 : 626, 1998*].

On la retrouve chez tous les mammifères [« Fibrillin, A new 350-kD Glycoprotein, is a Component of Extracellular Microfibrils » *Lynn Y. Sakai and all ; The Journal of Cell Biology, Vol. 103 (N°6, Pt.1) Dec. 1986 2499-2509*] mais aussi chez les invertébrés comme les holothuries [« Morphology and biomechamiscs of the microfibrillar network of the sea cucumber dermis » *Thurmond F.A. and all ; J. Exp. Biol., 199, 1817-1828*] et les méduses [« An Extracellular matrix protein of jellyfish homologous to mammalian fibrillins forms different fibrils depending on the life stage of the animal » *Reber-Muller S. and all ; Dev. Biol., 169, 662-672*].

La fibrilline possède une structure et des fonctions qui semblent s'être remarquablement conservées tout au long de l'évolution. Ainsi les fibrillines de méduse et de l'homme présentent une très forte homologie [« An Extracellular matrix protein of jellyfish homologous to mammalian fibrillins forms different fibrils depending on the life stage of the animal » *Reber-Muller S. and all ; Dev. Biol., 169, 662-672*].

La fibrilline permet à l'architecture des papilles dermiques de se maintenir, voire de se restaurer en cas de problème [« Age-related changes in the temporal and spatial distributions of fibrillin and elastin mRNAs and proteins in cutaneous wounds of healthy humans » *Ashcroft GS ; J. Pathol, 1997 Sept*.].

Sa structure et sa synthèse sont altérées par les U.V [« UVB irradiation alters cellular responses to cytokines rôle in extracellular matrix gene expression » *Werth VP ; J. Invest Dermatol., 1997 Mar*.].

La fibrilline n'a jamais été utilisée en cosmétique et en usage pharmaceutique.

Le brevet US-A-5648061 décrit un modèle de photo vieillissement utilisant des souris transgéniques chez lesquelles l'expression génétique de l'élastine et de la fibrilline est augmentée. Ce modèle n'utilise donc pas directement la fibrilline mais insiste sur son importance en tant que marqueur du photo vieillissement.

On sait que les cnidaires sont des organismes relativement simples, du type métazoaire, contenant essentiellement de l'eau et du collagène, dont les méduses sont la phase principale. La composition en acides aminés du collagène des méduses a été analysée [voir par exemple S. Kimura et al., "Le collagène comme principal constituant comestible de la méduse", J. of Food Science, vol 48, pp. 1758-1760 (1983)].

L'utilisation du collagène de cnidaires, ou de méduses, dans des compositions cosmétiques a été décrit dans le brevet FR-A-2714063.

Or, il a été constaté, à la suite des travaux réalisés par les inventeurs, qu'il était également possible d'obtenir de la fibrilline à partir des méduses utilisées pour préparer le collagène.

La présente invention a donc pour objet l'utilisation de la fibrilline comme constituant actif dans des compositions cosmétiques et pharmaceutiques, notamment cosmétologiques, pour le traitement et la prévention des affections de la peau.

L'invention a aussi pour objet un procédé de préparation de la fibrilline utilisable dans l'industrie des produits cosmétiques et pharmaceutiques, à partir de cnidaires, permettant d'obtenir une fibrilline de bonne qualité.

L'invention a également pour objet la fibrilline obtenue par ledit procédé, présentant une composition déterminée en acides aminés, son application en cosmétique, ainsi que des compositions cosmétiques et pharmaceutiques la contenant.

Suivant la présente invention, le procédé de préparation de fibrilline utilisable dans des compositions pharmaceutiques et cosmétiques, consiste à effectuer un lavage de cnidaires, suivi d'une extraction neutre, d'une séparation de phase, et d'une précipitation saline.

Il est préférable de traiter des cnidaires préalablement congelés, que l'on broie avant et/ou après l'étape de lavage.

L'étape d'extraction s'effectue en milieu neutre, par exemple en milieu faiblement salin de pH compris entre 5 et 8, suffisant pour mettre en suspension les glycoprotéines de structures. La séparation de phase peut s'effectuer par exemple en procédant par filtration grossière sur toile puis, si nécessaire, par centrifugation à basse vitesse (inférieure à 2.000 trs/min).

L'étape de précipitation saline, après séparation de phase suivant une technique classique, peut s'effectuer par exemple au moyen d'une solution aqueuse de chlorure de sodium, en concentration appropriée, de telle sorte que la force ionique du milieu soit comprise entre 1 et 4.

Il est préférable, conformément à l'invention, que la température ne dépasse pas 15°C environ dans les extracteurs, afin d'éviter une dénaturation des glyco-protéines de structures.

Le procédé conforme à la présente invention présente l'avantage d'être utilisable directement à l'échelle industrielle. Il permet de traiter simplement, par lots ou en continu, des quantités importantes de méduses, pouvant aller de quelques dizaines à plusieurs centaines de kilogrammes. Ainsi, à partir d'environ 600 kg de méduses, on peut préparer près de 2 à 5 kg de fibres essorées contenant les glycoprotéines de type fibrilline.

Le matériel fibreux est rincé puis décontaminé par de l'alcool de degré alcoolique compris entre 60 et 90 %, par exemple de l'alcool à 70 %, pendant environ 1 à 5 heures. Les suspensions de fibrilline sont ensuite préparées à partir de ce matériel fibreux à l'aide d'eau déminéralisée.

De plus, le procédé de l'invention utilise des cnidaires entiers, notamment des méduses, et non des sous-produits ou des fragments d'organismes.

La fibrilline obtenue conformément à la présente invention se distingue en ce que sa composition en acides aminés est comparable à celle de la fibrilline de vertébrés. Elle comprend au moins 0,5 % de cystine. De préférence, la teneur en cystine est comprise entre 0,5 % et 4,0 %.

On peut identifier la fibrilline de la présente invention par immunomarquage à l'aide d'un anticorps antifibrilline (technique dot-blots) et au microscope électronique à balayage où elle apparaît sous forme de fibres perlées de structure linéaire avec des globules de 20 à 25 nm de diamètre, caractéristiques de la fibrilline.

Les essais de tolérance effectués avec la fibrilline de l'invention sur les flancs rasés et préparés de lapins albinos, suivant une méthode usuelle, ont montré un indice d'irritation cutanée primaire inférieur à 0,4, significatif de l'absence d'irritation cutanée. De même, les essais d'irritation oculaire ont montré que la fibrilline de l'invention est très faiblement irritante (indice d'irritation maximale à 1 heure égal à 7,6).

Des études effectuées sur des fibroblastes de peau humaine ont montré que la fibrilline de l'invention n'est pas cytotoxique, et favorise l'activité mitochondriale et la prolifération cellulaire.

Les compositions cosmétiques préparées conformément à la présente invention ont fait apparaître d'intéressantes propriétés, notamment en ce qui concerne l'effet de stimulation de la cicatrisation des plaies, de régénération des tissus, d'assouplissement et d'adoucissement de la peau. De plus, la fibrilline présente des propriétés filmogènes et hydratantes.

Il a été également rapporté que ces compositions cosmétiques amélioraient les rides et les vergetures. La peau, ainsi traitée est également plus ferme.

Ces compositions cosmétiques contenant de la fibrilline peuvent, grâce à cette dernière, protéger la fibrilline naturellement présente dans le derme de l'utilisateur des effets délétères des U.V par un effet de leurre. En effet la fibrilline, ainsi interposée entre la source UV et les microfibrilles cutanées prend à son compte les photons destructeurs qui, ainsi stoppés, ne vont plus pouvoir atteindre leur cible dermique.

Une émulsion peut également libérer la fibrilline qu'elle contient qui devient disponible pour les protéases endogènes. Ces protéases en hydrolysant la fibrilline vont petit à petit libérer les motifs EGF dont cette dernière est constituée. Ces facteurs de croissance ainsi libérés à la demande vont stimuler le processus naturel du renouvellement cellulaire dermique et épidermique. Ce processus peut être particulièrement utile en cas de cicatrisation ou de brûlure, moment où la peau lésée libère beaucoup de protéase et où elle a besoin d'une augmentation de la prolifération cellulaire. De plus, en régulant le TGF β, la fibrilline contribue à améliorer la cicatrisation.

Les compositions à base de fibrilline suivant l'invention peuvent être présentées sous les formes usuelles de crème, émulsions, pommades ou gels.

La fibrilline selon l'invention s'applique non seulement dans le domaine des compositions cosmétiques, mais aussi dans celui des compositions pharmaceutiques où elle peut être utilisée isolément ou en association avec du collagène, notamment pour le traitement de patients atteints ou susceptibles de développer un syndrome de Marfan, et pour permettre la libération progressive de facteur de croissance du type EGF (Epidermal Growth Factor).

Suivant les techniques classiques, ces compositions peuvent contenir des excipients et supports usuels et peuvent se présenter sous forme de crème, de gel, d'émulsion, de pommade ou de patch à appliquer sur la peau, ou sous forme de comprimé ou de gélule destinés à être utilisés per os.

Les exemples suivants, donnés à titre non limitatifs, décrivent la préparation de fibrilline conformément à l'invention, ainsi que des compositions adaptées à une utilisation en cosmétique.

### Exemple 1

On concasse 600 kg de méduses congelées, que l'on laisse reposer en chambre froide (4°C) jusqu'à décongélation complète.

Après lavage à l'eau, on procède à un broyage puis à une extraction en milieu salin neutre (chlorure de sodium 0,15 M - hydrogénophosphate disodique 0,01M - Triton X 100 0,2 % - pH 7,4) dans un extracteur de 5m³.

L'extraction est réalisée pendant environ 1 heure, sous faible agitation au moyen d'un agitateur rotatif (50 trs/min) à une température comprise entre 12 et 15°C.

Après séparation de phase (élimination de débris...) la suspension de biomolécules est précipitée par addition de chlorure de sodium.

Les fibres de fibrillines précipitées en milieu salin (force ionique 2) sont essorées pour obtenir 2 à 5 kg de fibres à 5 % de protéines.

Après rinçage des fibres à l'alcool (éthanol à 70 % - 2 heures), les suspensions aqueuses dosées à 0,1 %, 0,2 % ou plus (0,6 %) de fibrillines sont préparées à l'aide d'eau déminéralisée.

On obtient ainsi un extrait de fibrillines se présentant sous forme de liquide visqueux légèrement opalescent, miscible à l'eau, de pH voisin de 4,0.

La composition en acides aminés des glycoprotéines de structure de type fibrilline de la fibrilline suivant l'invention est la suivante, comparée à celle de la fibrilline des vertébrés.

| | Fibrilline suivant l'invention | Fibrilline de vertébrés |
|---|---|---|
| Acide aspartique | 10,60 | 12,87 |
| Thréonine | 4,92 | 6,48 |
| Sérine | 6,50 | 7,23 |
| Acide glutamique | 10,04 | 12,01 |
| Proline | 8,90 | 7,38 |
| Glycine | 16,00 | 12,73 |
| Alanine | 10,11 | 4,23 |
| Valine | 2,35 | 4,15 |
| Cystine | 3,18 | 3,94 |
| Méthionine | 1,14 | 1,38 |
| Isoleucine | 2,87 | 5,43 |
| Leucine | 5,14 | 4,21 |
| Tyrosine | 1,69 | 3,40 |
| Phénylalanine | 2,79 | 3,25 |
| Histidine | 0,78 | 1,44 |
| Lysine | 4,37 | 3,32 |
| Arginine | 5,14 | 5,30 |
| Hydroxyproline | 0 | 0 |
| Hydroxylysine | 1,44 | Non déterminé |

L'analyse de la composition en acides aminés a été effectuée par chromatographie sur colonne échangeuse d'ions de la protéine hydrolysée, suivant une technique classique.

Les résultats de l'analyse indiqués ci-dessus montrent que la fibrilline obtenue suivant la présente invention contient environ 3 % de cystine.

La présence de cystine est particulièrement intéressante, conformément à l'invention, car elle améliore considérablement la qualité cosmétologique des compositions cosmétiques préparées avec la fibrilline de l'invention en raison de l'affinité de la cystine pour la kératine de la peau.

### Exemple 2

On prépare une émulsion huile dans eau en mélangeant les composants suivants, suivant la technique usuelle :

| | |
|---|---|
| Propylène glycol | 2,0 g |
| PEG 400 | 3,0 g |
| Conservateur | 0,3 g |
| Carbopol 941 | 0,2 g |
| Myristate d'isopropyle | 1,0 g |
| Alcool cétylique | 3,0 g |
| Acide stéarique | 3,0 g |
| Huile de germe de maïs | 2,0 g |
| Parfum | 0,5 g |
| Fibrilline de l'invention (à 0,6 %) | 4,0 g |
| Eau déminéralisée, quantité suffisante pour | 100,0 g |

On utilise la fibrilline en solution aqueuse à 0,6 %.

### Exemple 3

On prépare une émulsion eau dans huile en mélangeant les composants indiqués ci-après, suivant la technique usuelle :

| | |
|---|---|
| Protegin | 19,0 g |
| Huile de vaseline | 11,0 g |
| Glycérine | 4,0 g |
| Sulfate de magnésium | 0,5 g |
| Parfum | 0,6 g |
| Conservateur | 0,2 g |
| Fibrilline de l'invention (à 0,6 %) | 2,0 g |
| Eau déminéralisée, quantité suffisante pour | 100,0 g |

On utilise la fibrilline en solution aqueuse à 0,6 %.

### Exemple 4

On prépare un gel hydro-alcoolique en mélangeant les composants suivants , suivant la technique usuelle :

| | |
|---|---|
| Carbopol 941 | 1,0 g |
| Triéthanolamine | 1,0 g |
| Ethanol à 95 % | 60,0 g |
| Glycérol | 3,0 g |
| Propylène glycol | 1,0 g |
| Collagène de méduse (à 0,6 %) | 1,0 g |
| Fibrilline de l'invention (à 0,6 %) | 1,0 g |
| Eau déminéralisée, quantité suffisante pour | 100,0 g |

On utilise la fibrilline et le collagène issus tous les deux de méduse.

## Revendications

1. Procédé de préparation de fibrilline comprenant au moins 0,5% de cystine, utilisable dans des compositions pharmaceutiques et cosmétiques, ou comme biomatériau, **caractérisé en ce qu'**il consiste à effectuer un lavage de cnidaires, suivi d'une extraction neutre, d'une séparation de phase, et d'une précipitation saline.

2. Procédé selon la revendication 1, **caractérisé en ce que** l'on traite des cnidaires, notamment des méduses, préalablement congelées, que l'on broie avant et/au après l'étape de lavage.

3. Procédé selon la revendication 1, **caractérisé en ce que** l'extraction neutre est réalisée en milieu faiblement salin à un pH compris entre 5 et 8.

4. Procédé salon la revendication 1, **caractérisé en ce que** la précipitation saline est réalisée au moyen d'une solution aqueuse de chlorure de sodium, en concentration telle que la force ionique du milieu soit comprise entre 1 et 4.

5. Composition cosmétique ou pharmaceutique **caractérisée en ce qu'**elle comprend une fibrilline dont la composition en acides aminés comprend au moins 0,5 % de cystine.

6. Composition selon la revendication 5, **caractérisée en ce que** la teneur en cystine est comprise entre 0,5 % et 4,0†%.

7. Composition cosmétique selon l'une quelconque des revendications 5 et 6, **caractérisée en ce que** la fibrilline est associée à un support acceptable en cosmétique.

8. Composition cosmétique selon la revendication 7, **caractérisée en ce qu'**elle est sous forme de crème, de gel, d'émulsion ou de pommade.

9. Composition pharmaceutique selon l'une quelconque des revendications 5 et 6, **caractérisée en ce que** la fibrilline est associée à un support acceptable en pharmacie.

10. Composition pharmaceutique selon la revendication 9, **caractérisée en ce qu'**elle est sous forme de crème, de gel, d'émulsion, de pommade ou de patch à appliquer sur la peau ou sous forme de comprimé ou de gélule destinés à être utilisés per os.

11. Composition cosmétique ou pharmaceutique selon l'une quelconque des revendications 7 à 10, **caractérisée en ce qu'**elle associe du collagène et de la fibrilline,

12. Utilisation de la fibrilline dont la composition en acides aminés comprend au moins 0,5 % de cystine pour la préparation de compositions cosmétiques et pharmaceutiques pour le traitement et la prévention des affections de la peau.

## Claims

1. Method of preparing fibrillin comprising at least 0.5 % cystine, usable in pharmaceutical and cosmetic compositions or as a bio-material, **characterised in that** it consists in carrying out washing of cnidaria, followed by neutral extraction, separation of phase, and saline precipitation.

2. Method according to claim 1, **characterised in that** cnidaria, in particular jellyfish, are processed which have been previously frozen and are pulverised before and/or after the washing stage.

3. Method according to claim 1, **characterised in that** neutral extraction is carried out in a slightly saline medium with a pH of between 5 and 8.

4. Method according to claim 1, **characterised in that** saline precipitation is carried out by means of an aqueous solution of sodium chloride in a concentration such that the ionic strength of the medium is between 1 and 4.

5. Cosmetic or pharmaceutical composition **characterised in that** it comprises a fibrillin whose amino acid composition includes at least 0.5 % cystine.

6. Composition according to claim 5, **characterised in that** the content of cystine is between 0.5 % and 4.0 %.

7. Cosmetic composition according to either of claims 5 or 6, **characterised in that** the fibrillin is combined with a support which is cosmetically acceptable.

8. Cosmetic composition according to claim 7, **characterised in that** it takes the form of a cream, gel, emulsion or ointment.

9. Pharmaceutical composition according to either of claims 5 or 6, **characterised in that** the fibrillin is combined with a support which is acceptable as a pharmaceutical.

10. Pharmaceutical composition according to claim 9, **characterised in that** it takes the form of cream, gel, emulsion, ointment or a patch to be applied to the skin or the form of a tablet or capsule intended to be taken orally.

11. Cosmetic or pharmaceutical composition according to any one of claims 7 to 10, **characterised in that** it combines collagen and fibrillin.

12. Use of fibrillin, whose amino acid composition includes at least 0.5 % cystine, for preparing cosmetic and pharmaceutical compositions for the treatment and prevention of skin diseases.

## Patentansprüche

1. Verfahren zur Herstellung von Fibrillin mit einem Cystingehalt von zumindest 0,5 %, das in pharmazeutischen und kosmetischen Zusammensetzungen oder als Biomaterial einsetzbar ist, **dadurch gekennzeichnet, dass** das Verfahren darin besteht, Waschen von Cnidaria vorzunehmen, gefolgt von Neutralextraktion, Phasentrennung und Aussalzung.

2. Verfahren nach Anspruch 1, **dadurch gekennzeichnet, dass** Cnidaria, insbesondere Quallen/Medusen, behandelt werden, die vorzugsweise vorher tiefgefroren wurden und vor und/oder nach dem Schritt des Waschens gemahlen werden.

3. Verfahren nach Anspruch 1, **dadurch gekennzeichnet, dass** die Neutralextraktion in einem schwach salzhaltigen Medium bei einem pH zwischen 5 und 8 durchgeführt wird.

4. Verfahren nach Anspruch 1, **dadurch gekennzeichnet, dass** die Aussalzung mit einer wässrigen Natriumchloridlösung in einer solchen Konzentration durchgeführt wird, dass die lonenstärke des Mediums zwischen 1 und 4 liegt.

5. Kosmetische oder pharmazeutische Zusammensetzung, die **dadurch gekennzeichnet ist, dass** sie ein Fibrillin enthält, dessen Aminosäurenzusammensetzung zumindest 0,5 % Cystin umfasst.

6. Zusammensetzung nach Anspruch 5, **dadurch gekennzeichnet, dass** der Cystingehalt zwischen 0,5 und 4,0 % liegt.

7. Kosmetische Zusammensetzung nach einem der Ansprüche 5 oder 6, **dadurch gekennzeichnet, dass** das Fibrillin mit einem kosmetisch annehmbaren Träger assoziiert ist.

8. Kosmetische Zusammensetzung nach Anspruch 7, **dadurch gekennzeichnet, dass** sie in Form einer Creme, eines Gels, einer Emulsion oder einer Salbe vorliegt.

9. Pharmazeutische Zusammensetzung nach einem der Ansprüche 5 oder 6, **dadurch gekennzeichnet, dass** das Fibrillin mit einem pharmazeutisch annehmbaren Träger assoziiert ist.

10. Pharmazeutische Zusammensetzung nach Anspruch 9, **dadurch gekennzeichnet, dass** sie in Form einer Creme, eines Gels, einer Emulsion, einer Salbe oder eines Pflasters zum Aufbringen auf die Haut oder in Pastillen- oder Gelkapselform zur oralen Verwendung vorliegt.

11. Kosmetische oder pharmazeutische Zusammensetzung nach einem der Ansprüche 7 bis 10, **dadurch gekennzeichnet, dass** sie Kollagen und Fibrillin miteinander assoziiert.

12. Einsatz von Fibrillin, dessen Aminosäurenzusammensetzung zumindest 0,5 % Cystin enthält, für die Herstellung kosmetischer und pharmazeutischer Zusammensetzungen zur Behandlung von und Vorbeugung gegen Hauterkrankungen.
